# EUROPEAN PATENT APPLICATION

(11) **EP 1 800 612 A1**
(43) Date of publication of application: **27.06.2007**
(21) Application number: 06256224.4
(22) Date of filing: 06.12.2006
(51) Int. Cl.: A61B 17/22, A61B 18/14, A61B 18/00, A61M 1/00

(54) **Improvements in or relating to surgical instruments**

(30) Priority: 22.12.2005 GB 0526151
(71) Applicant: Eschmann Holdings Limited, Lancing, West Sussex BN15 8TJ (GB)
(72) Inventor: Gonon, Bertrand, 69370 Ternay (FR)
(74) Representative: Peel, James Peter

(57) **Abstract**

The invention provides a surgical dissection instrument comprising an electrode suitable for use in electrosurgery and a nozzle for use in generating a pressure jet of liquid wherein the electrode has a proximal end for connection to an electrosurgery generator and an electrode tip at the distal end and wherein the nozzle has a proximal end for connection to a source of pressurised liquid and a distal end at which the pressure jet is generated; and a method of using the instrument.

To be accompanied, when published, by Figure 1 of the drawings.

## Description

The present invention relates to a combined electrosurgery and water-jet hand piece.

During surgery, a surgeon carries out three principal procedures or interventions: dissection, cutting and coagulation. These three procedures are generally used for about 80% of the duration of a typical surgical intervention. Most of the time, a surgeon uses the dissection procedure with the rest of the time split between cutting and coagulation.

According to traditional practice, a surgeon will use a water-jet hand piece, a CUSA (a Cavitron ultrasonic surgical aspirator) or scissors and grips for dissection. For cutting, a surgeon will use scissors or an electrosurgery unit. For coagulation, a surgeon will use thread, staples or an electrosurgery unit.

New surgical instruments have recently been launched which provide combined functionality. For example Johnson & Johnson have a Harmonic Scalpel (registered trade mark) which is an ultrasonic cutting and coagulation device; Valleylab sell their Ligasure (registered trademark) system which is a combined grip and vessel sealing generator; and TissueLink market their FB (registered trade mark) floating ball system which provides coagulation with a fluid delivery system. Thus these new devices, although claiming to have combined functionality, in fact are able to perform only two of the above-noted surgical procedures that may be required during surgery.

A problem therefore remains with the currently available multifunction surgical instruments in that they fail to provide a surgeon with a single instrument that is capable of performing at least all three of the above-noted typical surgical procedures.

According to the invention there is provided a surgical instrument adapted to be used to perform at a given time any one of a plurality of surgical procedures which instrument comprises an electrode suitable for use in electrosurgery and a nozzle for generating a pressure jet of liquid wherein the electrode has a proximal end for connection to an electrosurgery generator and an electrode tip at the distal end and wherein the nozzle has a proximal end for connection to a source of pressurised fluid and a distal end at which the pressure jet is output and wherein the electrode and the nozzle are selectively actuable to perform respective functions to effect different surgical procedures.

The advantages of the instrument according to the invention include that it provides a single instrument which can be used in the three interventions identified above which are dissection, cutting and coagulation. This is because the pressure jet of fluid generated by the nozzle can be used in a dissection intervention and the electrode can be used in a cutting or coagulation intervention. The fluid used in the invention is preferably a liquid, more preferably water, especially an aqueous saline solution.

It should be understood that the term "electrode" is intended to cover any electrically conducting implement which is suitable for conducting high frequency electricity. The electrode is generally formed from a material which is physiologically acceptable, for example stainless steel. For example, the electrode could be in the form of a conventional surgical instrument such as forceps. The electrode tip is optionally in the form of a bar, blade, hook or a different form commonly used in the art.

The nozzle and electrode of the instrument according to the invention are preferably separate. The advantage of such as an arrangement is that the nozzle and electrode may be moved in relation to eachother. The nozzle is preferably insulated from the pressure jet in the instrument according to the invention. This is useful as a safeguard to protect the patient.

The instrument according to the invention preferably has an electrode which is moveable relative to the instrument such that the electrode tip can be extended from the instrument in use or retracted to protect the electrode tip. Preferably the instrument has a mechanism which can be operated to extend the electrode tip from the instrument which is generally referred to herein as a switch mechanism. The electrode is preferably moveable mechanically or magnetically. The electrode or switch may be arranged so that the electrode is biased to a retracted position. Alternatively, the switch may be biased to an extended position and provided with a mechanism to lock it in a retracted position. The switch preferably has a first position wherein the electrode is caused to extend from the instrument and a second position where the electrode is caused to be retracted in the instrument. The switch preferably has a third position wherein the electrode is caused to be retracted and wherein in use the source of pressurised liquid is caused to operate such that the nozzle generates a pressure jet of liquid.

The switch preferably has a pressure jet switch mechanism which is operated when the switch is moved to the third position. The pressure jet switch mechanism is optionally an electrical contact switch mechanism or a pneumatic discontinuity switch mechanism. The pneumatic discontinuity switch mechanism has a tube which is connected, in use, to a source of vacuum or of pressurised gas via a sensor which detects flow rate, pressure or vacuum discontinuity. When pressure jet switch mechanism preferably is a pneumatic discontinuity switch mechanism, it is operated as follows: when the switch is moved to the third position to operate the pressure jet switch mechanism, the switch preferably blocks the tube so as to generate a pressure or vacuum discontinuity which is detected by the sensor. The sensor then causes the source of pressurised liquid to operate. The pneumatic discontinuity switch mechanism is preferably substantially as described in US 6 508 823, the contents of which are incorporated herein by reference, especially as described with reference to Figures 7 and 8 of the drawings of that patent.

The electrode is preferably supported by a tube and can be moved within the tube.

The instrument according to the invention preferably comprises a tube having a first lumen which forms the nozzle and a second lumen which supports the electrode wherein the electrode is moveable within the second lumen. Each lumen is preferably in the form of an axial chamber in the tube. The tube optionally has one or more additional lumens which could be used for providing suction at a location where the instrument is used or for delivering a medicament to the location.

Alternatively, where the electrode is not moveable itself, the tip of the electrode may preferably be protected by a cover or shroud which is moveable relative to the electrode so that the electrode tip can be protected when it is not in use.

The instrument according to the invention preferably has a nozzle which comprises a hollow tube having an axial lumen wherein the lumen has a restriction at the distal end in which an orifice is formed. The orifice in the restriction preferably has an axial length (which is the length of the restriction) and a diameter and wherein ratio of the axial length of the orifice to its diameter is from 1:1 to 5:1. The nozzle preferably has an additional lumen which is preferably arranged such that the axes of the two lumen are substantially parallel.

The instrument preferably has a control panel for controlling the characteristics of the electrical supply to the electrode, e.g. the current frequency, polarity, voltage etc. The control panel preferably has one or more buttons by which the supply may be controlled. For example the control panel may have two buttons, one of which, when operated, causes the supply to provide continuous high frequency electricity suitable for use in a cutting procedure and the other of which, when operated, causes the supply to provide pulsed high frequency electricity suitable for use in a coagulation procedure. The control panel is preferably concealed when the electrode is not in use. Preferably the control panel is concealed by the switching mechanism used to extend the electrode tip.

According to the invention there is also provided a method of performing surgery on a patient in need of such treatment which method comprises the steps of
providing a surgical dissection instrument as defined in any one of the preceding claims; and, either
actuating the instrument to provide electrical power to the electrode and performing a cutting or coagulation intervention; or
actuating the instrument to provide a pressure jet of liquid and performing a dissection intervention.

The nozzle used in the invention is preferably formed from an insulating plastics material which is preferably a plastics material having mechanical strength, purity, chemical resistance, ease of processing (including mouldability) and sterilization resistance. The plastic material is more preferably a thermoplastic polymeric material, especially a thermoplastic polycondensate. Examples of suitable plastic materials include a polyimide, polycarbonate, polyetheretherketone, polyaryletherketone, polyphenylene oxide, polysulfone and/or a polyphenylene sulphide. Most preferably the plastic material is a polyaryletherketone resin.

The invention will now be illustrated, by way of example, with reference to the Figures of the accompanying drawings in which:
**Figure 1** shows a schematic cross-sectional view of a surgical dissection instrument according to a first embodiment of the invention;
**Figure 2** shows a schematic cross-sectional view of a first nozzle for use in the surgical dissection instrument according to the first or second embodiment;
**Figure 3** shows a schematic cross-sectional view of a surgical dissection instrument according to a second embodiment of the invention;
**Figure 4** shows a schematic cross-sectional view of a surgical dissection instrument according to a third embodiment of the invention;
**Figure 5** shows a schematic cross-sectional view of a second nozzle for use in the surgical dissection instrument according to the third embodiment;
**Figure 6** shows a schematic cross-sectional view of a third nozzle for use in the surgical dissection instrument according to the third embodiment;
**Figure 7** is a cut-out perspective view of a first embodiment of a switch mechanism for use in the surgical dissection instrument of the third embodiment of the invention;
**Figure 8** is a schematic cross-sectional view of a second embodiment of a switch mechanism for use in the surgical dissection instrument of the third embodiment of the invention which shows the mechanism in a first position;
**Figure 9** is a schematic cross-sectional view of the second embodiment of a switch mechanism for use in the surgical dissection instrument of the third embodiment of the invention which shows the mechanism in a second position;
**Figure 10** is a schematic cross-sectional view of a fourth nozzle for use in the surgical dissection instrument according to the third embodiment;
**Figure 11A** is a schematic cross-sectional view of a third embodiment of a switch mechanism for use in the surgical dissection instrument of the third embodiment of the invention which shows the mechanism in a first position;
**Figure 11B** is a schematic cross-sectional view of the third
embodiment of a switch mechanism for use in the surgical dissection instrument of the third embodiment of the invention which shows the mechanism in a second position;
**Figure 11C** is a schematic cross-sectional view of the second embodiment of a switch mechanism for use in the surgical dissection instrument of the third embodiment of the invention which shows the mechanism in a third position; and
**Figure 12** is a schematic cross-sectional view of a fifth nozzle for use in the surgical dissection instrument according to the third embodiment.

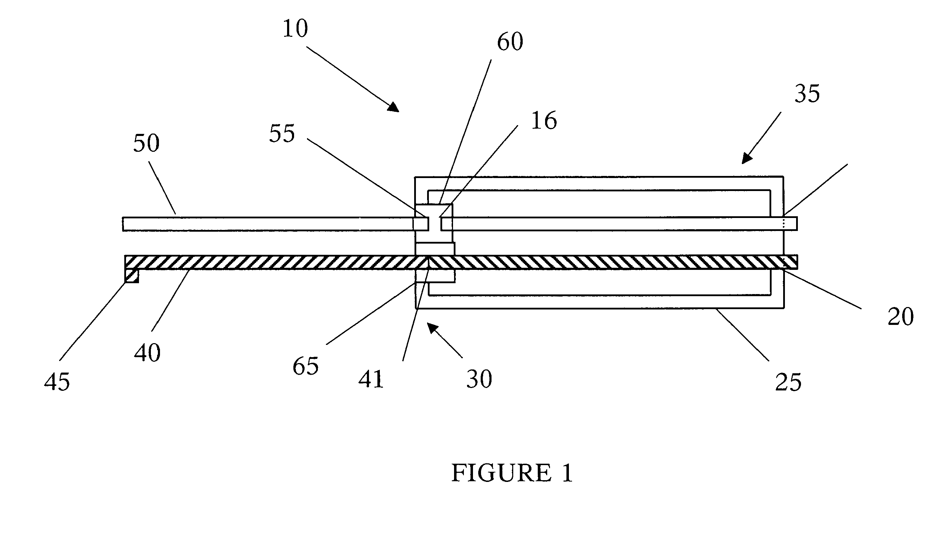

With reference to Figure 1, a first embodiment of a surgical dissection instrument according to the invention comprises a handpiece 10, a first flexible tube 15 for connection, in use, to a source (not shown) for supplying a high-pressure physiological salt solution and a flexible wire 20 for connection, in use, to an output of an electrosurgery generator (not shown). The first flexible tube 15 is typically reinforced by a reinforcement made of braided synthetic wires, so as to be able to withstand the high pressure of the liquid, which may reach 70 bar.

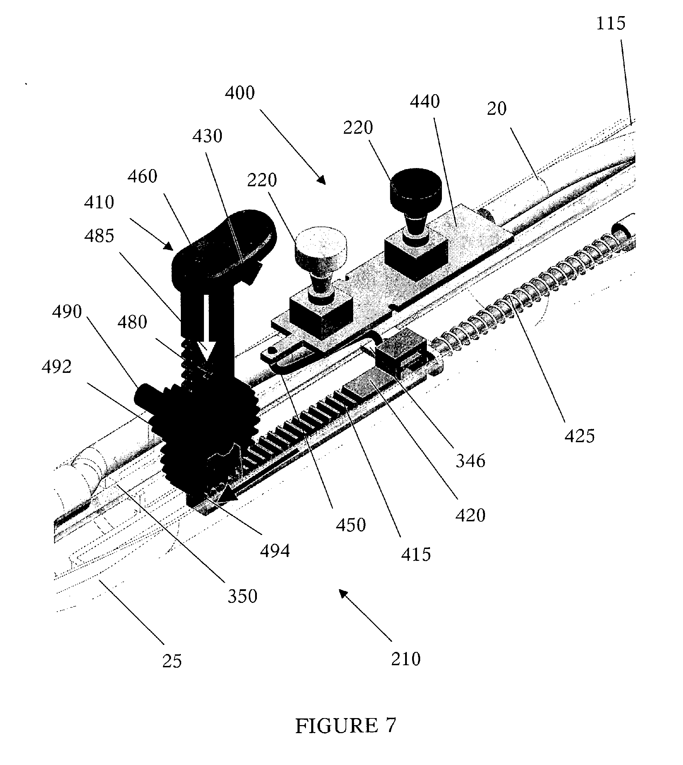

The control means (not shown) for the source (not shown) for supplying a high-pressure physiological salt solution and for the electrosurgery generator (not shown) is arranged so as not to supply the high-pressure physiological salt solution at the same time as electrical power from the electrosurgery generator. The control means (not shown) may optionally be provided on handpiece 10 or separately. The source for supplying a high-pressure physiological salt solution is optionally arranged to supply a flow of droplets of physiological salt solution when the electrosurgery generator is operating. In order that the source for supplying a high-pressure physiological salt solution can operate in this way, the source is optionally a bi-functional generator as described in US 6 083 189, the contents of which are incorporated herein by reference. The electrosurgery generator is optionally a TD830 electrosurgery unit manufactured by Eschmann Equipment. This electrosurgery unit is suitable for use with the invention because it can function in a mono- or di- polar mode. The control means may be a control panel 440 described with reference to Figure 7 which control means may be provided on handpiece 10 or separately as a handheld control means or foot operated control means.

The handpiece 10 is of a disposable type, made entirely from injection-moulded thermoplastic synthetic material. It primarily comprises an ergonomic body 25 of generally elongated shape, with a substantially circular cross-section, this body extending from a distal end 30 to a proximal end 35, both ends 30,35 being substantially located on the same axis. The proximal end 35 incorporates an opening through which the tube 15 and wire 20 enter the body 25.

The ergonomic body 25 is made up of two substantially identical moulded half shells. The two half shells are hollow on the inside to allow the tube 15 and wire 20 to pass inside the body of the handpiece 10 from the proximal end 35 to the distal end 30 along a substantially straight path.

An elongate nozzle 50 is securely fitted to the first tube 15. The distal end 16 of first tube 15 and the proximal end 55 of the nozzle 50 are respectively securely fitted to a respective side of an annular collar 60 of plastic material, for example by push fitting the respective ends 16,55 of the tube 15 and the nozzle 50 into respective annular ends of the collar 60 and then bonding the assembly with adhesive or by thermal bonding.

An elongate electrode 40 formed from conductive material is securely connected to wire 20 by annular collar 65. The distal end of wire 20 is soldered or otherwise electrically connected to the proximal end 41 of the electrode 40 and then the connection is reinforced by collar 65. Electrode 40 has a tip 45 at its distal end.

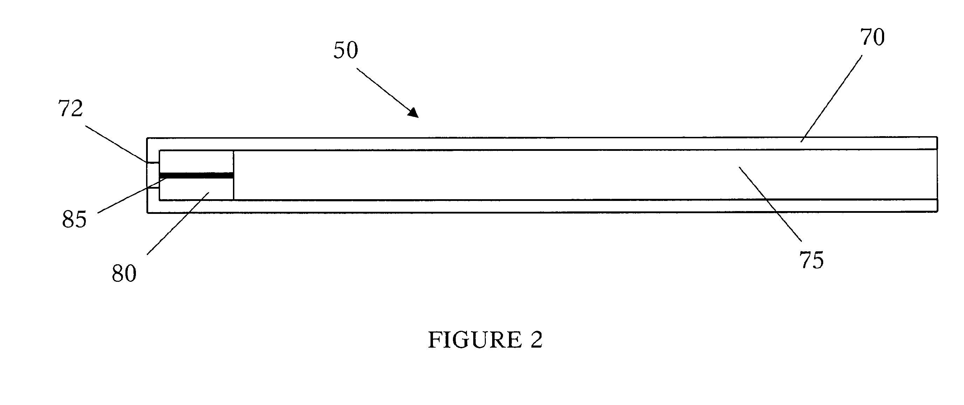

The nozzle 50 is shown in more detail in Figure 2. It comprises a cylindrical tube 70 defining an axially directed central bore or lumen 75 extending therealong. The tube 70 is formed from metal such as stainless steel. Tube 70 has at its distal end a restriction 80 which has an orifice 85. The restriction 80 is formed from metal. As an alternative, a semi-precious material such as sapphire or a plastics material could be used. The nozzle 50 is formed by inserting the restriction 80 into tube 70 and then closing the end 72 of tube 70 to seal in the restriction 80.

The orifice 85 is an axially directed central orifice which extends through the restriction 80. The restriction 80 typically has an axial length of from 0.1, preferably from 0.3mm to 2mm, preferably to 1 mm, more preferably to 0.5mm typically approximately 0.8 mm or about 0.4mm, with the orifice 60 having the same axial length. Thus the orifice is an elongated orifice. The diameter of the bore 75, and therefore the diameter of the proximal face of the restriction 80 subjected to liquid pressure in the bore 75 and the internal diameter of the tube 50, is typically from 1.5 to 2.5 mm, more typically approximately 1.7 mm. The internal diameter of the orifice 85 is typically from 0.05 to 0.4 mm, more typically approximately 0.2 mm (e.g. about 0.22mm). The ratio of the axial length to the internal diameter is from 1:1, preferably from 1.2:1, more preferably from 1.3:1 to 5:1, preferably to 4.5:1, more preferably to 4:1. Preferably the ratio is about 1.5:1 or about 3.7:1. This structure of the restriction 80 is sufficient to withstand typical liquid pressures in the nozzle 50 of up to 70 bar without failure or deformation of the restriction 80.

The external diameter of the tube 70 is typically from 2 to 4 mm, more typically approximately 2.3 mm, thereby giving a typical wall thickness of from 0.5 to 2 mm, more typically approximately 0.6 mm.

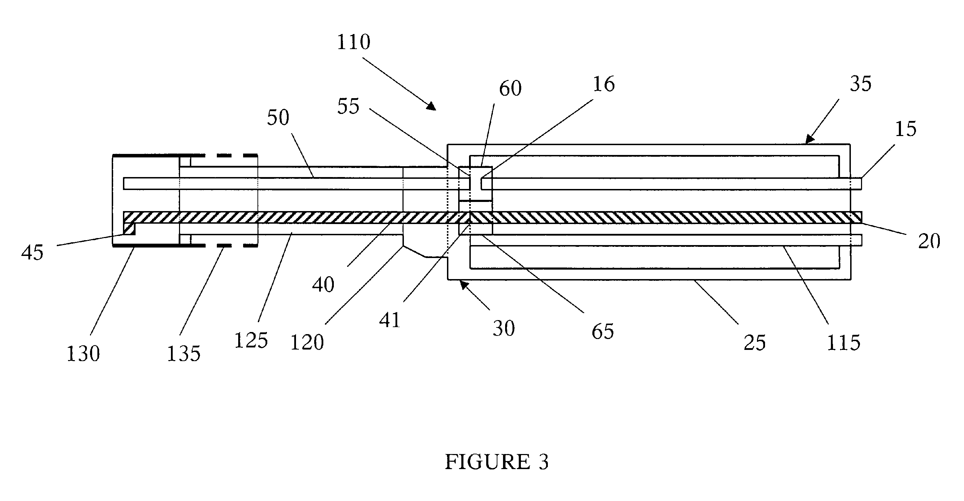

As an alternative, nozzle 50 may be formed from a plastics material. A suitable plastics material for use in the construction of tube 70 and/or restriction 80 is typically composed of a thermoplastic polymeric material, especially a thermoplastic polycondensate such as a polyimide, polycarbonate, polyetheretherketone, polyaryletherketone, polyphenylene oxide, polysulfone and/or polyphenylene sulphide. Most preferably it is composed of a polyaryletherketone resin, which exhibits mechanical strength, purity, chemical resistance, ease of processing and sterilization resistance. Nozzle 50 may be a nozzle substantially as described with reference to Figure 3 of co-pending patent application no. PCT/GB2005/000553; the contents of this document are incorporated herein by reference.

As an alternative to the first embodiment illustrated in Figure 1, the handpiece 10 may comprise an elongate return electrode in addition to the electrode 40 such that the handpiece may act as a bi-polar electrosurgery unit. The return electrode may be provided parallel to the electrode 40 at a sufficient distance for it to act in this manner.

The handpiece 110 of the second embodiment of a hand-operated surgical dissection instrument of the present invention is illustrated in Figure 3. Like features of the two embodiments are identified by like identification numerals. Handpiece 110 is of similar construction to handpiece 10, having an ergonomic body 25 made up of two substantially identical moulded half shells.

Handpiece 110 has a manifold 120 which is bonded in a fluid-tight manner to the distal end 30 of the ergonomic body 25. The manifold 120 defines an internal chamber which is in fluid communication with a second flexible tube 115 which, in use, is connected to a source of suction. An aspirator tube 125 of plastic material is fitted to the manifold 120, by being push fitted into the manifold 70. The aspirator tube 82 is cylindrical and surrounds the nozzle 50 and the electrode 40. The distal end of the aspirator tube 125 is located a small distance, about 2 to 4 mm or less, proximally of the distal end of the nozzle 50 and of the electrode tip 45. Therefore the distal end of the nozzle 50 and the electrode tip 45 protrude from the distal end of the aspirator tube 125. The distal end of the nozzle 50 and the electrode tip 45 are protected by a shroud 130. Shroud 130 is movable from a distal position where it protects the distal end of the nozzle 50 and the electrode tip 45 to a proximal position shown at 135 where the distal end of the nozzle 50 and the electrode tip 45 are exposed such that the electrode tip 45 is available for use.

The distal end of the aspirator tube 125 is provided with at least one vent hole (not shown) extending through the wall thickness thereof. In the illustrated embodiment there are two diametrically opposed circular vent holes (not shown), each of a diameter of about 1 to 1.5 mm. The at least one vent hole is provided to obviate the aspirator tube 125 from inadvertently attaching itself to the patient's body under the negative pressure applied to the aspirator tube 125.

The aspirator tube 125 is typically composed of polyvinylchloride and typically has a wall thickness of from 0.25 to 1.0 mm so as to be flexible, with typically the inner diameter being from 3.5 to 5 mm and the outer diameter being from 4 to 6 mm. The aspirator tube 125 is preferably transparent so that a user can readily check that it has not become inadvertently blocked in use.

The control means (not shown) for the source for supplying a high-pressure physiological salt solution and for the electrosurgery generator is arranged so as not to supply the high-pressure physiological salt solution at the same time as electrical current from the electrosurgery generator. The control means (not shown) may optionally be provided on handpiece 110 or separately.

As an alternative to the second embodiment illustrated in Figure 3, the handpiece 110 may comprise an elongate return electrode in addition to the electrode 40 such that the handpiece may act as a bi-polar electrosurgery unit. The return electrode may be provided within aspirator tube 125 parallel to the electrode 40 at a sufficient distance for it to act in this manner.

As a further alternative, the aspirator tube 125 is constructed from a physiologically acceptable rigid material such as metal so that the handpiece can be used in laparoscopic surgery. The aspirator tube 125 may optionally be provided with a physiologically acceptable coating, e.g. a phospholipid.

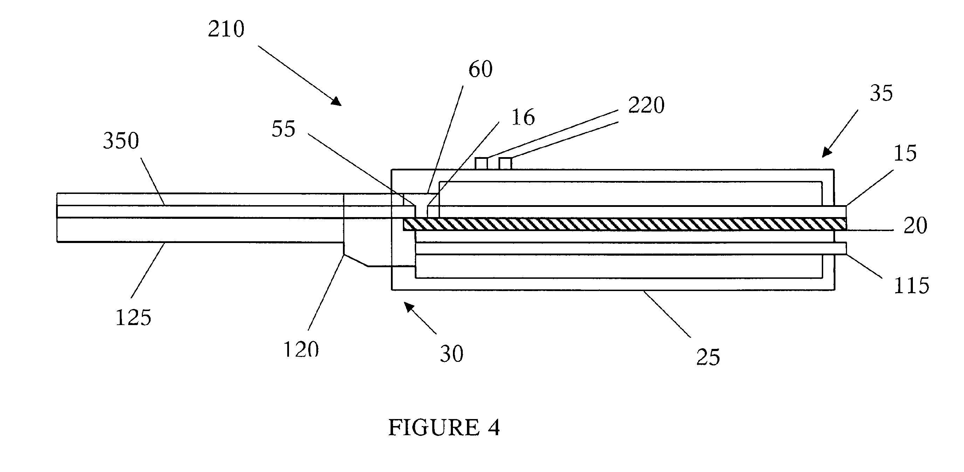

The handpiece 210 of the third embodiment of a hand-operated surgical dissection instrument of the present invention is illustrated in Figures 4 and 7. Like features of the three embodiments are identified by like identification numerals. Handpiece 210 is of similar construction to handpiece 10, having an ergonomic body 25 made up of two substantially identical moulded half shells.

The handpiece 210 has a first flexible tube 15, a flexible wire 20 and a second flexible tube 115. The electrosurgical action of the electrode is controlled by buttons 220 on the handpiece 210.

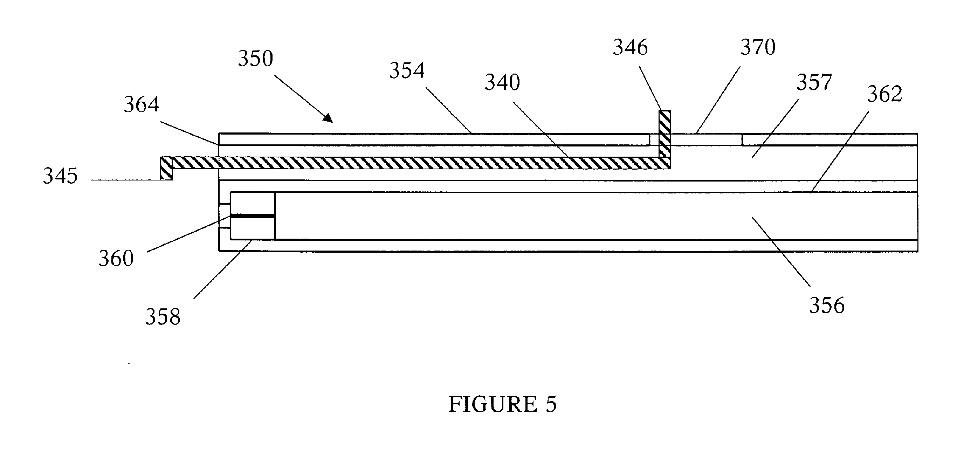

Handpiece 210 comprises a nozzle 350 formed from a plastics material as defined above in relation to nozzle 50. Nozzle 350 is shown in detail in Figure 5. Nozzle 350 is provided in the form of a cylindrical tube 354 having two substantially parallel lumen or bores 356,357 separated by a web 362. Lumen 357 terminates at the distal end of the nozzle 350 with an orifice 364. Lumen 357 contains an electrode 340 having an electrode tip 345. Part 346 of the electrode 340 protrudes from a longitudinal opening 370 in lumen 357. The opening 370 is arranged so that part 346 can be moved in a proximal direction such that the electrode 340 can be withdrawn into lumen 357 through orifice 364. Nozzle 350 may be a nozzle substantially as described with reference to Figure 5 of co-pending patent application no. PCT/GB2005/000553.

Lumen 356 terminates at the distal end of the nozzle 350 with a restriction 358 having an axially directed central orifice 360 extending through it. Restriction 358 is formed from metal. As an alternative, a semi-precious material such as sapphire or a plastics material could be used.

Lumen 356 of nozzle 350 is securely fitted to the first tube 13 and part 346 of the electrode 340 is connected to wire 20. Annular collar 60 is used to secure the connections.

The movement of the electrode 340 in and out of lumen 357 of nozzle 350 is controlled by a switch mechanism 400 which is shown in Figure 7. Switch mechanism 400 has an electronic control panel 440 that may be in the form of a printed circuit (PC) board onto which switches 220 are connected, for example by being solder-jointed. Switches 220 are for controlling the polarity of the electrical power supplied to the electrode. The control panel 440 has a further nozzle control switch (not shown) for controlling the operation of the source of high pressure physiological salt solution. Control panel 440 has an input from wire 20 and is connected to part 346 of the electrode 340 by wire 450, both of which are also connected for example by being solder-jointed to the printed circuit board. Control panel 440 further has logic control means (either hard wired logic including for example an Exclusive Or gate or a microprocessor electrically coupled to the printed control board) which prevents nozzle control switch (not shown) from switching on the source of high pressure physiological salt solution (not shown) when the electricity is being supplied to the electrode 345 or when the electrode 345 is extended from the handpiece 210. Of course, the converse is true in that when pressurised fluid is being output from the nozzle, the electrode 345 is prevented from being actuated or powered on. As an alternative embodiment, control panel 440 may be omitted and replaced with foot operated switch or hand controlled switch to provide the same function.

Switch mechanism 400 has a first actuator indicated generally at 410 which is formed from a high impact plastics material. The first actuator 410 is T-shaped with the vertical part of the first actuator 410 elongated compared to the transverse part. The upper surface of the transverse part of the first actuator 410 is formed as a control surface or button 460. On the underside of the transverse part of the button 460, a catch 430 is provided.

The actuator 410 is moveable from a first to a second position. Catch 430 engages with an opening 26 in the housing 25 to lock the actuator 410 in the second position. On the vertical part of the first actuator 410, a toothed portion 480 is provided.

Switch mechanism 400 has a second actuator 420 which is bonded to part 346 of electrode 340. The second actuator 420 is also formed from a high impact plastics material and is moveable from a first position wherein the electrode 340 is retracted to a second position where the electrode is extended. The second actuator 420 is connected to a resilient member 425 which is shown in the form of a coiled spring. Resilient member 425 is bonded to the housing 25. Resilient member 425 biases the second actuator towards the first position. The second actuator has a toothed portion 415.

Switch mechanism 400 has two toothed wheels 492,494 which are mounted on axle 490 which is itself mounted on the housing 25. First toothed wheel 492 has a smaller diameter than second toothed wheel 494. First wheel 492 engages with the toothed portion 480 of the first actuator 410. Second wheel 494 engages with the toothed portion 415 of the second actuator 420.

Vertical downward movement 485 of the first actuator 410 causes the toothed portion 480 to engage and move the first toothed wheel 492. Movement of the first toothed wheel 492 causes the axle 490 to turn second toothed wheel 494. Second toothed wheel 494 then engages and moves the toothed portion 415 of the second actuator 420. Thus vertical movement 485 of the first actuator 410 is converted into transverse movement of the second actuator 420 and thus of electrode 340. This movement continues until catch 430 engages in opening 26 and locks the first actuator 410 and second actuator 420 in their respective second positions such that the electrode is extended.

Release of the catch 430 from opening 26 causes the electrode 340 to be retracted because resilient member 425 causes the second actuator 420 to return to its first position and in so doing, causes the first actuator 410 to return to its first position as well.

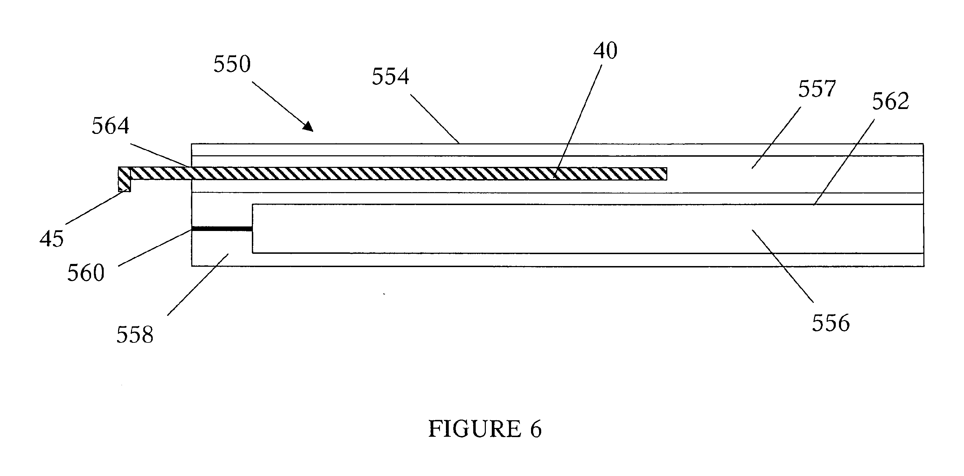

As an alternative to the mechanism shown in Figure 7, the movement of the electrode could be controlled by a magnet moveable by means of switch from a first position to a second position and biased to return to a first position. Such a mechanism could be substantially as shown in Figure 7 but with the mechanism providing linear movement of a magnet. In such an embodiment, nozzle 350 could be replaced by nozzle 550 which is a nozzle according to a third embodiment of the present invention and which is shown in Figure 6. Nozzle 550 is provided in the form of a cylindrical tube 554 having two substantially parallel lumen or bores 556,557 separated by a web 562. Lumen 557 terminates at the distal end of the nozzle 550 with an orifice 564. Lumen 556 terminates at the distal end of the nozzle 550 with an integral restriction 558 having an axially directed central orifice 560 extending through it. The length of the integral restriction 558 and the width of the orifice 560 are substantially the same as the restriction 80 and orifice 85 for the first embodiment of the nozzle according to the invention. Lumen 557 contains an electrode 40 having an electrode tip 45. Nozzle 550 may be a nozzle substantially as described with reference to Figure 5 of co-pending patent application no. PCT/GB2005/000553.

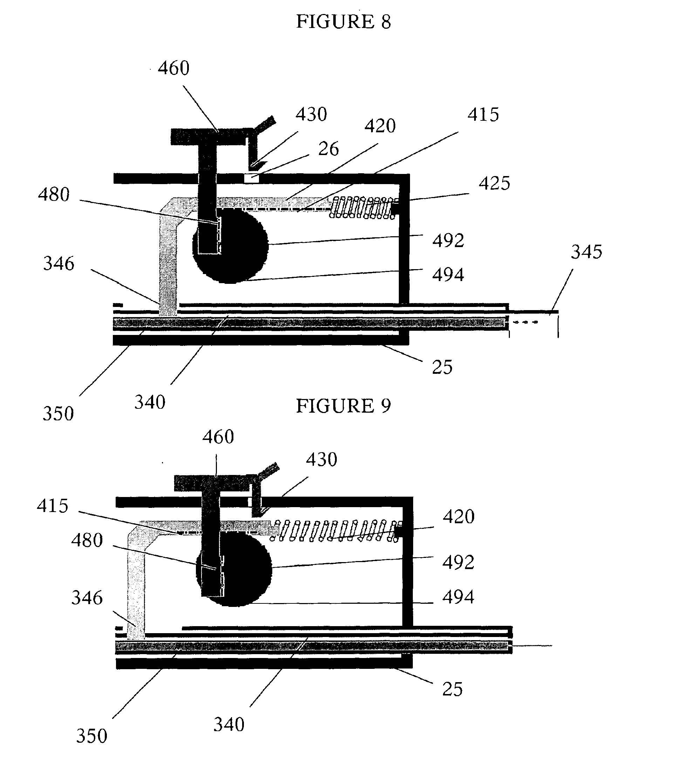

As a further alternative to the switch mechanism shown in Figure 7, the second embodiment of a switch mechanism shown in Figures 8 and 9 could be used. In this embodiment, like features to those of the first embodiment of a switch mechanism shown in Figure 7 have like reference numerals. The second embodiment of a switch mechanism functions in the same manner as the first embodiment except that in the first position, the electrode 345 is extended and in the second position (where the catch 430 engages with an opening 26 in the housing 25 to lock the actuator 410 in the second position), the electrode 345 is retracted.

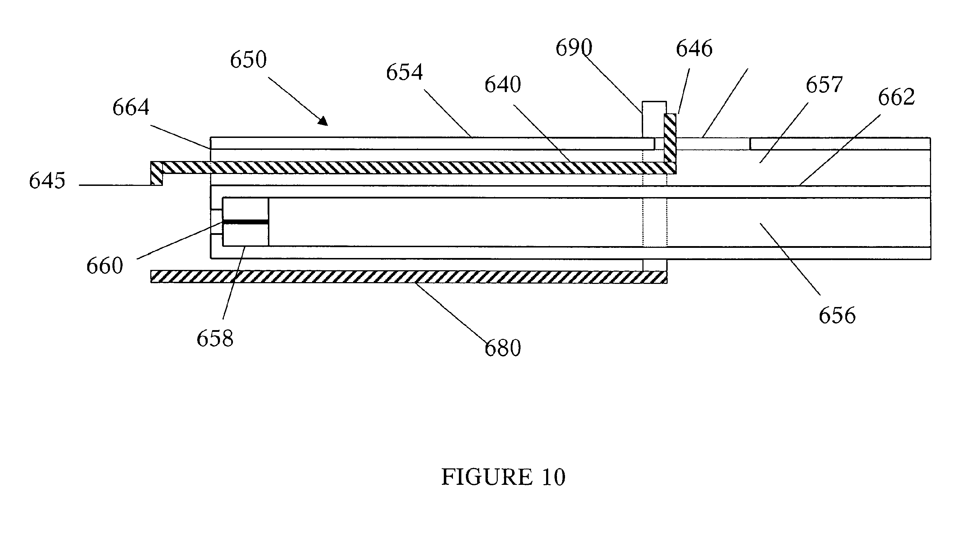

As a further alternative to nozzle 350 in the third embodiment, nozzle 650 shown in Figure 10 may be used. Nozzle 650 is provided in the form of a cylindrical tube 654 having two substantially parallel lumen or bores 656,657 separated by a web 662. Lumen 657 terminates at the distal end of the nozzle 650 with an orifice 664. Lumen 656, used for generating a pressure jet of fluid, terminates at the distal end of the nozzle 650 with an integral restriction 658 having an axially directed central orifice 660 extending through it. The length of the integral restriction 658 and the width of the orifice 660 are substantially the same as the restriction 80 and orifice 85 for the first embodiment of the nozzle according to the invention. Lumen 657 contains an electrode 640 having an electrode tip 645. Part 646 of the electrode 640 protrudes from a longitudinal opening 670 in lumen 657. The opening 670 is arranged so that part 646 can be moved in a proximal direction such that the electrode 640 can be withdrawn into lumen 657 through orifice 664. Nozzle 650 may be a nozzle substantially as described with reference to Figure 5 of co-pending patent application no. PCT/GB2005/000553. Nozzle 650 has a return electrode 680 mounted on its external surface such that the instrument which comprises nozzle 650 can be used in bi-polar surgery. So that the return electrode 680 and the electrode 640 can be extended at the same time, they are connected by member 690 which is formed from an insulating plastics material. In particular, part 646 of electrode 640 is connected to member 690. Member 690 is adapted to be connected to the switch mechanism (not shown).

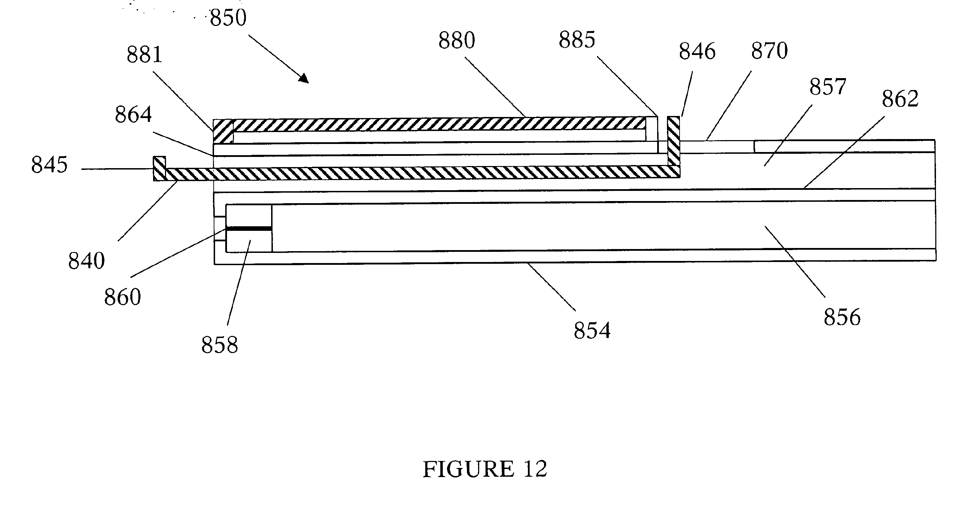

As a further alternative to nozzle 350 in the third embodiment, nozzle 850 shown in Figure 12 may be used. Nozzle 850 is provided in the form of a cylindrical tube 854 having two substantially parallel lumen or bores 856,857 separated by a web 862. Lumen 857 terminates at the distal end of the nozzle 850 with an orifice 864. Lumen 856, used for generating a pressure jet of fluid, terminates at the distal end of the nozzle 850 with an integral restriction 858 having an axially directed central orifice 860 extending through it. The length of the integral restriction 858 and the width of the orifice 860 are substantially the same as the restriction 80 and orifice 85 for the first embodiment of the nozzle according to the invention. Lumen 857 contains an electrode 840 having an electrode tip 845. Part 846 of the electrode 840 protrudes from a longitudinal opening 870 in lumen 857. The opening 870 is arranged so that part 846 can be moved in a proximal direction such that the electrode 840 can be withdrawn into lumen 857 through orifice 864. Nozzle 850 may be a nozzle substantially as described with reference to Figure 5 of co-pending patent application no. PCT/GB2005/000553. Nozzle 850 has a return electrode 880 mounted on its external surface such that the instrument which comprises nozzle 850 can be used in bi-polar surgery. Return electrode 880 is mounted on insulating housing 885 and is fixed in position. Return electrode 880 presents a return electrode surface 881 which is flush with the distal end of the nozzle 850. The return electrode 880 is mounted on the side of the nozzle 850 which is proximate to electrode 840 such that movement of electrode tip 845 relative to return electrode surface 881 allows an operator of an instrument comprising nozzle 850 and having a suitable control mechanism (not shown) for controlling the movement of electrode 840 to grip tissue between electrode tip 845 and return electrode surface 881. Thus the combination of electrode tip 845 and return electrode surface 881 act as forceps and may be used in electrosurgical techniques such as fusing tissue.

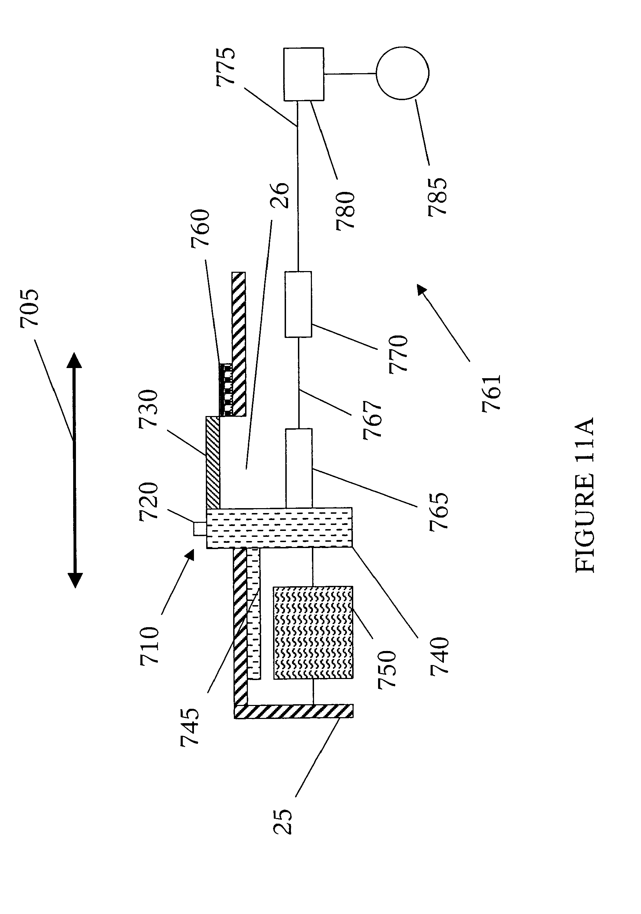

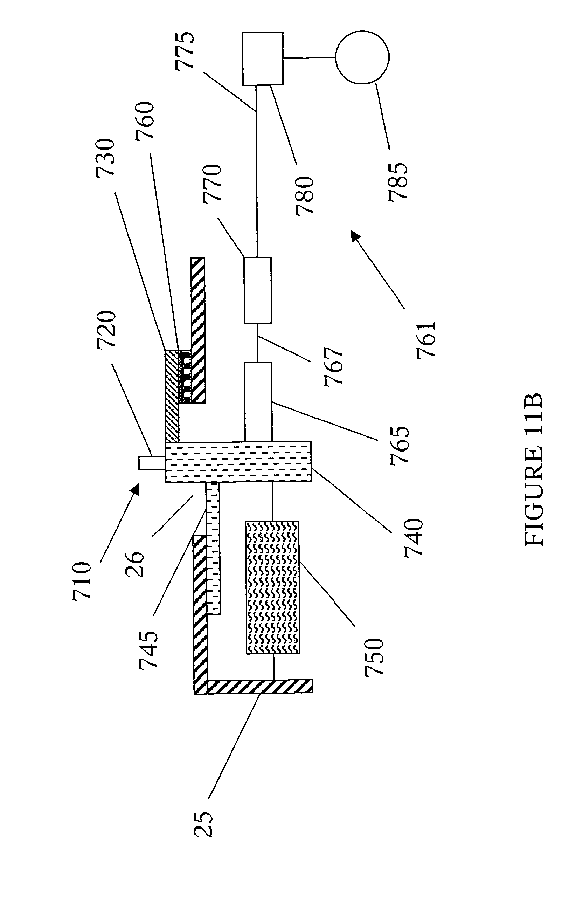

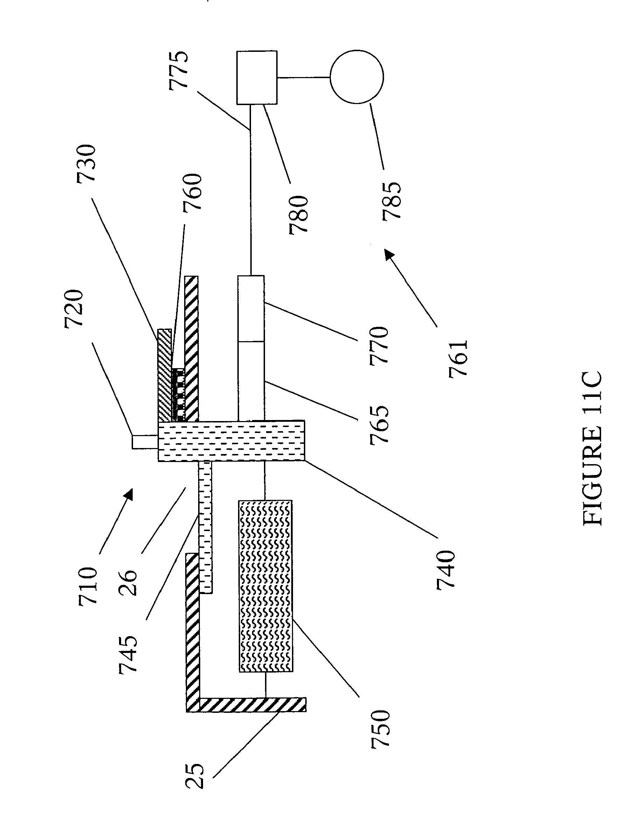

A third switch mechanism 710 for use in the third embodiment of the invention is shown in Figures 11A, 11B and 11C. This switch mechanism can be used as an alternative to the switch mechanisms illustrated in Figures 7-9. Switch mechanism 710 is moveable in a longitudinal direction shown by arrow 705 between three positions which are a first position where the electrode(s) 40,680 is/are extended from the instrument, illustrated in Figure 11A; a second position where the electrode(s) 40,680 is/are retracted into the instrument, illustrated in Figure 11B; and, a third position where the electrode(s) 40,680 is/are retracted into the instrument and the switch mechanism causes the operation of the source for supplying a high-pressure physiological salt solution, illustrated in Figure 11C.

Switch mechanism 710 has a body 740 which is connected (not shown) to the electrode 40 or the member 690 such that movement of the switch mechanism 710 moves the electrode(s) 40,680. The switch mechanism 710 has a blocking member 745 which blocks the opening 26 into the housing 25 when the switch mechanism 710 is in the second and third positions illustrated in Figures 11B and 11C. To ensure the proper functioning of switch mechanism 710, housing 25 forms a channel (not shown) through which blocking member 745 slides. Switch mechanism 710 has a cover 730 which blocks the opening 26 in housing 25 when the switch mechanism 710 is in the first or second positions illustrated in Figures 11A and 11B.

Switch mechanism 710 is connected to the housing 25 by means of resilient member 750 which is in the form of a spring. Resilient member 750 is arranged so as to bias the switch mechanism to the first position shown in Figure 11A.

Switch mechanism 710 has a spring-loaded button 720 which is moveable between a locked position shown in Figures 11B and 11C and an open position shown in Figure 11A. In the locked position, the resilient member 750 is prevented from moving the switch mechanism to the first position. However, the switch mechanism is still able to move from the second to the third position. Depression of the button 220 in the locked position whilst the switch mechanism is in the second or third position causes the resilient member to move the switch mechanism to the first position.

The housing 25 has a control panel 760 positioned adjacent the switch mechanism 710. Control panel 760 is used to control the power supply for the electrode(s) 40,680. Cover 730 protects the control panel 760 when the switch mechanism 710 is in the second and third positions and the electrode(s) 40,680 are retracted into the instrument. The control panel 760 may be a control panel 440 substantially as described with reference to Figure 7. As an alternative embodiment, control panel 760 may be omitted and replaced with foot operated switch or hand controlled switch to provide the same function.

In its third position, switch mechanism 710 causes the operation of the source for supplying a high-pressure physiological salt solution, illustrated in Figure 11C by means of pneumatic discontinuity switch mechanism 761. To operate pneumatic discontinuity switch mechanism 761, switch mechanism 710 has a plunger 767 mounted on guide tube 765 which is itself mounted on body 740. Pneumatic discontinuity switch mechanism 761 has a tube 770 into which plunger 767 fits. Tube 770 is connected to a vacuum source 785 via sensor 780 by line 775 such that, in operation, there is a flow of air through tube 770. Vacuum source 785 may be connected to a vacuum system commonly found in operation rooms of hospitals. As an alternative, source 785 may be a source of pressure. Sensor 780 detects flow rate, pressure or vacuum discontinuity in the line 775 and is a detector as shown in Figure 8 of US 6 508 823 and described at column 8 lines 8 to 32 of that patent. Sensor 780 is connected to the source for supplying a high-pressure physiological salt solution (not shown) and causes the operation of the source for supplying a high-pressure physiological salt solution when it detects a discontinuity.

In operation, when the switch mechanism 710 is moved to the third position shown in Figure 11C, plunger 767 is moved into tube 770 such that guide tube 765 closes tube 770. The flow of air through tube 770 is then prevented by guide tube 765 sealing tube 770. This pneumatic discontinuity is then detected by sensor 780 such that the source for supplying a high-pressure physiological salt solution is switched on.

With the inventive surgical instrument as described above, during surgery, instead of needing multiple devices, a surgeon now only needs to select the operative mode he requires at any given time. For example, when facing the need for a dissecting procedure, the surgeon would switch the instrument to the dissecting mode whereby pressurised fluid is output from the nozzle. On the other hand, when cutting is required, the surgeon could simply switch the unit to its cutting mode whereby power (continuous high frequency electricity) is provided to the electrode to effect cutting. Finally if a coagulation procedure is required, the instrument may be switched to its coagulating mode so that pulsed high frequency electricity is provided to the electrode to enable the surgeon to perform the coagulation procedure.

The present invention is not restricted to the forms of embodiment described, but can undergo various alterations and be presented in various aspects derived from the forms described in an obvious manner for a person skilled in the art. For instance, instead of being limited to supplying high frequency continuous or pulsed power to the electrode to effect cutting or coagulation, respectively, other forms of power (low frequency voltage for example) may also be provided to the electrode to effect other types of procedures, for example warming, that currently are not being utilised with an electrode device in surgery.

## Claims

1. A surgical instrument adapted to be used to perform at a given time any one of a plurality of surgical procedures which instrument comprises an electrode suitable for use in electrosurgery and a nozzle for generating a pressure jet of liquid wherein the electrode has a proximal end for connection to an electrosurgery generator and an electrode tip at the distal end and wherein the nozzle has a proximal end for connection to a source of pressurised fluid and a distal end at which the pressure jet is output and wherein the electrode and the nozzle are selectively actuable to perform respective functions to effect the different surgical procedures.

2. An instrument as defined in Claim 1 wherein the electrode is separate from the nozzle.

3. An instrument as defined in Claim 1 or Claim 2 wherein the electrode is insulated from the pressure jet.

4. An instrument as defined in any one of the preceding claims wherein the electrode is moveable relative to the instrument such that the electrode tip is extended from the instrument in use or retracted to protect the electrode tip and wherein the nozzle is not actuable when the electrode tip is extended from the instrument.

5. An instrument as defined in Claim 4 wherein the electrode is moveable mechanically.

6. An instrument as defined in Claim 4 or Claim 5 wherein the electrode is biased to an extended or a retracted position.

7. An instrument as defined in any one of the preceding claims wherein the electrode is supported by a tube and can be moved within the tube from an extended position to a retracted position.

8. An instrument as defined in any one of the preceding claims which comprises a tube having a first lumen which forms the nozzle and a second lumen which supports the electrode.

9. An instrument as defined in claim 8 wherein the electrode is moveable within the second lumen.

10. An instrument as defined in any one of the preceding claims further comprising a switch mechanism for selectively controlling the respective functions of the electrode and nozzle.

11. An instrument as defined in claim 10 wherein movement of the electrode is controlled by the switch mechanism which has a first position where the electrode is extended and a second position where the electrode is retracted.

12. An instrument as defined in claim 11 wherein the switch has a third position wherein the electrode is retracted and wherein in use the source of pressurised liquid is switched on such that the nozzle generates a pressure jet of liquid.

13. An instrument as defined in Claim 1 which has a part which is moveable relative to the electrode so that the electrode tip can be protected when it is not in use.

14. An instrument as defined in any one of the preceding claims wherein the nozzle comprises a hollow tube having an axial lumen wherein the lumen has a restriction at the distal end in which an orifice is formed.

15. An instrument as defined in Claim 14 wherein the orifice has an axial length and a width and wherein ratio of the axial length of the orifice to its width is from 1:1 to 5:1.

16. An instrument as defined in any one of the preceding claims which has a control panel for controlling the electrical supply to the electrode.

17. An instrument as defined in claim 16 wherein the control panel is concealed when the electrode is not in use.

18. An instrument as defined in any one of the preceding claims which comprises a return electrode such that it can act as a bi-polar electrosurgery unit.

19. An instrument as defined in claim 18 wherein the electrode tip is moveable in relation to the return electrode, preferably such that the electrode tip and return electrode act in combination as forceps.

20. An instrument as defined in any one of the preceding claims which has an aspirator tube which may be connected to a source of suction.

21. An instrument as defined in claim 20 wherein the aspirator tube supports the electrode and nozzle such that the instrument may be used in laparoscopic surgery.

22. An instrument as defined in any one of the preceding claims wherein the electrode is selectively actuable to perform a cutting procedure or a coagulation procedure and the nozzle is selectively actuable to perform a dissection procedure.
